# EUROPEAN PATENT SPECIFICATION

(11) **EP 3 377 155 B1**
(45) Date of publication and mention of the grant of the patent: **27.07.2022**
(21) Application number: 16863245.3
(22) Date of filing: 18.11.2016
(51) Int. Cl.: A61M 5/32, A61M 5/315, A61M 5/20, A61M 5/50, A61M 5/31

(54) **RETRACTABLE SYRINGE**
EINZIEHBARE SPRITZE
SERINGUE RÉTRACTABLE

(43) Date of publication of application: 26.09.2018
(73) Proprietor: Global Medisafe Holdings Ltd, Newcastle, NSW 2300 (AU)
(72) Inventor: WALSH, Allan, Medowie, New South Wales 2318 (AU)
(74) Representative: FRKelly
(86) International application number: PCT/AU2016/051119
(87) International publication number: WO 2017/079811

(56) References cited:
- WO-A1-00/48651
- WO-A1-02/11796
- WO-A1-2006/096909
- WO-A1-2008/009063
- WO-A1-2008/128274
- WO-A1-2013/083979
- US-A- 5 407 436
- US-A- 6 099 500
- US-A1- 2005 070 854
- US-A1- 2012 004 621

## Description

### Technical Field

The invention relates to the field of hypodermic syringe manufacture. In particular, the invention relates to a retractable safety syringe, in which the needle is capable of being withdrawn into the barrel of the syringe after use via a spring or similar mechanism.

### Background of the Invention

Safety syringes for hypodermic needles are becoming a very important tool of healthcare. The ability to prevent needle-stick injuries is extremely important in preventing the spread of blood-borne diseases.

One particular type of safety syringe is the retractable syringe. That is: a syringe wherein there is provided a mechanism, usually a spring, inside the syringe that is activated upon completion of the injection of the fluid, whereby the spring acts to retract the needle inside the syringe barrel. This prevents the needle from causing a needle-stick injury, and in particular it allows the needle operator to withdraw the needle via a one-handed operation, which makes it much easier to achieve in the situation of providing injections to patients in a busy hospital or clinic.

One type of retractable syringe is that disclosed in WO 2006/096901, wherein there is provided a syringe with a hollow plunger that can accommodate a withdrawn needle and needle hub, the hub and needle being actuated by the expansion of a spring into the plunger, and where the spring is released by pressure of the plunger on the assembly that holds the hub in place by restraining the spring from release.

Such syringes are referred to commonly as 'auto-retractable', meaning that the actuation of the needle retraction is provided by a mechanism in the syringe itself, as opposed to requiring the operator to pull, push or otherwise move the needle into the barrel of the syringe.

A drawback with this type of retractable syringe is that they are prone to liquid leakage, and the mechanism tends to suffer from functional reliability issues.

Other designs are relatively complex and so are difficult or expensive to manufacture. Yet other designs do not allow for full dispensing of the injectable fluid, due to dead-zones being left in the barrel.

Accordingly, it is an object of the invention to provide a retractable syringe construction that ameliorates at least some of the problems associated with the prior art.

### Summary of the Invention

According to a first aspect of the invention, there is provided an auto-retractable syringe as claimed in claim 1.

The detent arrangement that holds the needle hub more firmly in place, by disallowing movement in both directions, provides a particular advantage.

The claws have an inclined surface that is inclined away from the needle hub, such that when engaged by a member moving in the direction of injection, the force applied thereby will tend to push the claws away from the needle hub, thereby releasing it and allowing the biasing member to push the hub into the plunger. Such upper surfaces allow for a more reliable engagement of the claws, and allow simple engagement by another moving part to create a controlled disengagement of the claws.

Preferably, the plunger has at its liquid-engaging end a stopper, said stopper being held in place by a detent arrangement that may be overcome by the force of the biasing member pushing the needle hub into the plunger. This allows the plunger to be pushed back into the cavity along with the needle. It also makes the syringe easier to manufacture and allows a more efficiently shaped plunger stopper to be employed, such as one with a partly-spherical liquid-engaging surface.

In a preferred embodiment, the biasing member is a spring, and said spring is installed partly compressed. Partial compression of the spring (as opposed to full compression) allows for simpler construction and better control of the retraction operation.

Now will be described, by way of a specific, non-limiting example, a preferred embodiment of the invention with reference to the drawings.

### Brief Description of the Drawings

Figure 1 is a cross-sectional view of the 'needle' end of a syringe according to the invention.
Figure 2 is a cross-sectional view of a syringe according to the invention, shown prior to completion of dispensing.
Figure 3 is a cross-sectional view of the syringe of figure 4, shown at the point of completion of dispensing.
Figure 4 is a cross-sectional view of the syringe of figure 4, shown following retraction of the needle hub.

### Detailed Description of the invention

The invention may be embodied by the arrangement of working parts at the 'dispensing' end of a retractable hypodermic syringe. These are best understood by reference to the accompanying figures.

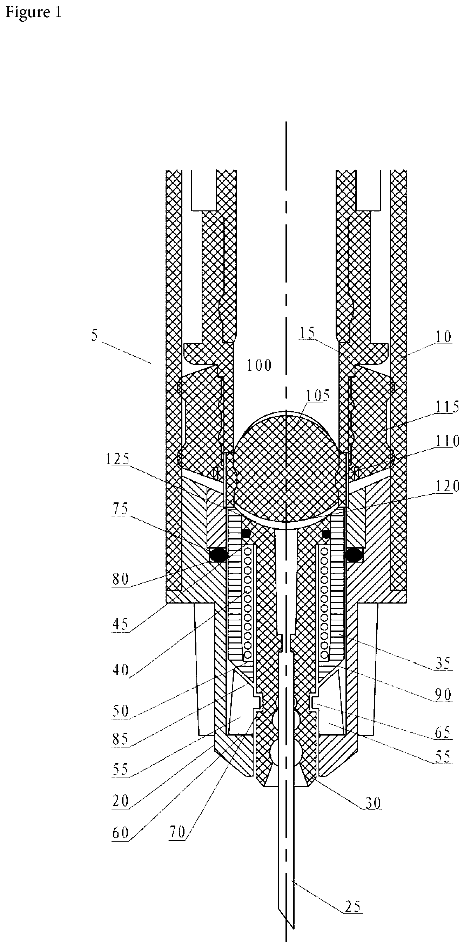

Turning to figure 1, there is shown an auto-retractable syringe 5. The major components of the syringe are the barrel 10, the plunger 15, the gland 20 and the needle 25. The gland 20 is inserted in the barrel 10 in a press-fit manner and houses the needle hub assembly. It is also possible for the gland to be a screw-in type.

The needle hub assembly includes the needle hub 30, in which is inserted the needle 25. A sliding activator sleeve 35 is fitted around the needle hub and in between these two parts is placed a partially compressed spring 40. The spring 40 is in contact with a stepped rear-facing surface 45 of the hub and an inner forward stepped surface 50 of the sleeve 35. Thus the spring's role is to tend to force the hub 30 rearward relative to the sleeve 35.

However, prior to dispensing, this tendency of the spring 40 is held in check by a detent mechanism. This mechanism consists of three resilient claws 55 that protrude from the inner front surface 60 of the gland 20. Each of said claws have a square or rectangular lug 65 oriented toward the needle hub 25. They also have an rearward 'head' surface 85 that is smooth but sloped in a sense that it is furthest from the surface of the hub 30 at its upper-most extremity, and said surface is closer to the hub 30 or the gland 20 at the 'dispending end' of the claw 55.

The activator sleeve 35 is chamfered around its forward edge to produce an angled surface 90. In assembly, this chamfered surface 90 is located as close as possible to the complementarily sloped 'head' surface 85 of the three claws 55. The closeness of the location means a more rapid response to dispensing pressure on the activation sleeve 35, as there is less distance to travel.

The needle hub 25 has a complementary groove 70 around its circumference that is located such that the lugs 65 are received within said groove 70 in pre-dispensing configuration. The positioning and shape of the lugs 65 and the groove 70 provide a very reliably immobile positioning of the needle hub, as the square shape of the lugs/groove combination prevents movement of the hub 30 in either a forward or rearward direction.

An O-ring 75 is located around the outer circumference of the activator sleeve 35 and positioned in an internal groove 80 in the inner surface of the gland 20. This provides a more effective seal around the hub/sleeve assembly to prevent fluid leakage during dispensing.

The plunger 15 is formed as a hollow barrel, with an internal cavity 100. The end of said plunger barrel is sealed via a stopper 105 made of rubber and which is held in place by a simple detent ring 110. On the outside of the plunger barrel is arranged a rubber gasket 115, which makes a sealing engagement with the inner surface of the barrel 10. The forward surface 120 of the stopper is partly spherical and the rear surface 125 of the needle hub 30 are shaped in a complementary manner to afford maximum dispensing and minimal 'dead zones'.

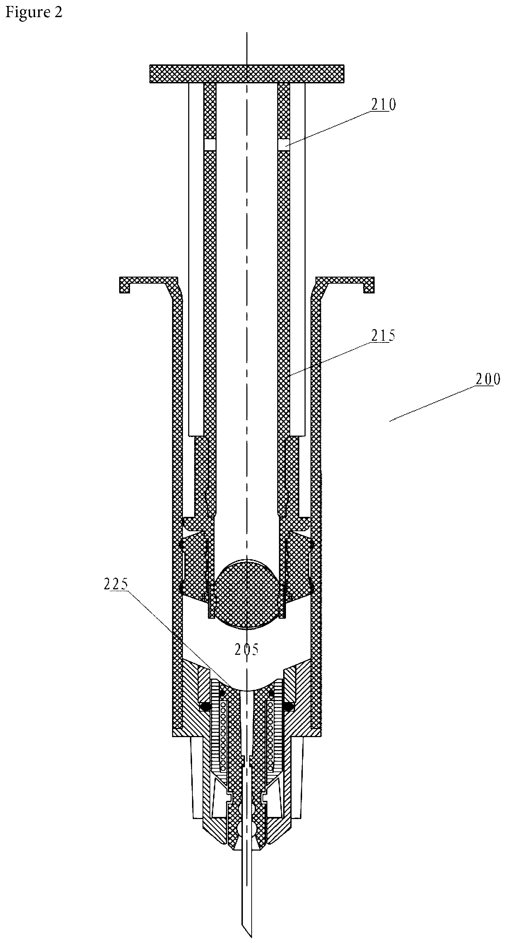

Turning to figure 2, there is shown a syringe 200 according to the invention during the fluid dispensing phase, where the fluid is in the cavity 205 formed between the plunger 215 and the rear of the needle hub 225. The remainder of the parts are as described in figure 1. In addition, it will be noted that there are provided 'air escape' holes 210 in the plunger barrel 215, which are required to allow air to escape as the hub is (later) pushed in to the cavity 205.

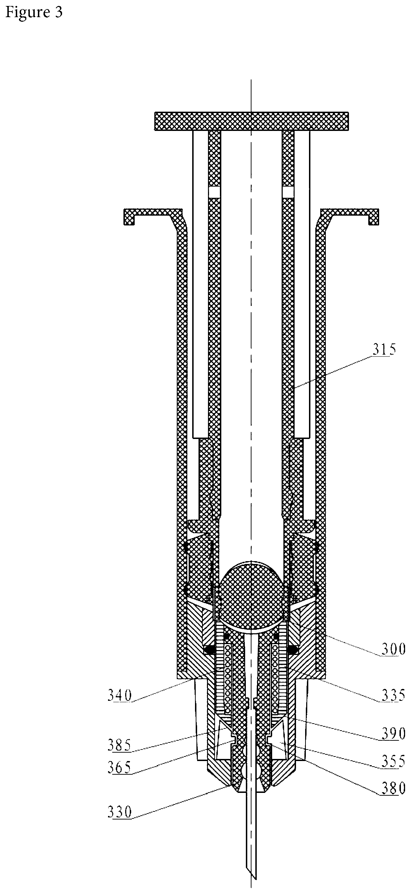

Turning to figure 3, there is shown the syringe of figure 2 at the point of full dispensing, just as pressure is brought to bear on the rear end 300 of the activation sleeve 335 by the plunger 315. As the activation sleeve 335 is pushed forward, the chamfered forward surface 390 can be seen making contact with the angled 'head' surface 385 of the claws 355 and beginning to force the claws away from the hub 330, thereby causing the lugs 365 to begin to disengage with the groove 380, thereby to release the spring 340.

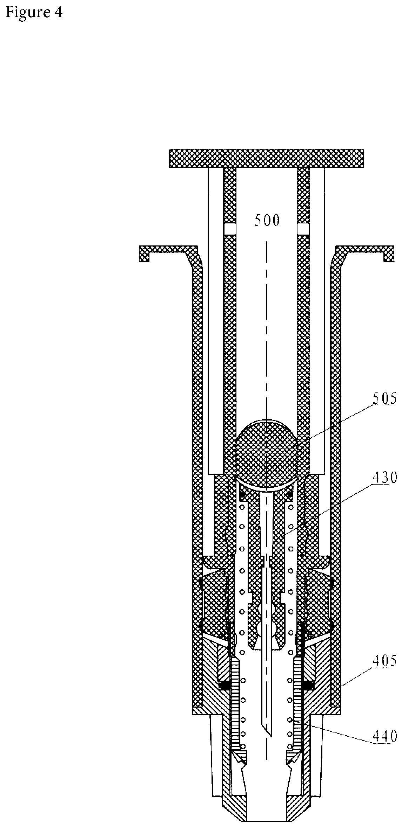

Turning to figure 4, there is shown the syringe of figure 3 post the release of the detent mechanism. The spring 440 has extended and thereby pushed the needle hub 430 and the stopper 505 well inside the plunger's internal cavity 500. The needle 425 can be seen to be also well inside the body of the syringe 405. At his point the syringe can be safely disposed of without risk of needle-stick injury.

It will be appreciated by those skilled in the art that the above described embodiment is merely one example of how the inventive concept can be implemented. It will be understood that other embodiments may be conceived that, while differing in their detail, nevertheless fall within the scope of the invention as determined by the claims.

## Claims

1. An auto-retractable syringe (5) having a barrel (10) including an open forepart, and a plunger (15) moveable within said barrel (10), a gland assembly (20) that is inserted, by press-fit or screw-in, into the open forepart of said barrel (10), said gland assembly (20) housing a removable needle hub (30) to which is attached a hypodermic needle (25); wherein a partly compressed spring (40) is disposed between the gland assembly (20) and said needle hub (30) so as to tend to urge the needle hub (30) into said barrel (10); wherein said plunger (15, 215) has an internal cavity capable of receiving the needle hub (30) and one or more holes (210) to allow displaced air to escape; wherein the needle hub (30) is kept in place prior to use by a detent arrangement that prevents movement of the needle hub (30) both into and out of the barrel (10); wherein the detent arrangement comprises one or more indent regions in the needle hub (30), said indent region or regions having a profile that, when engaged would prevent movement of said needle hub (30), whether into or out of the barrel (10); and a set of resilient claws (55) that have complementary lugs that occupy the indent regions, thereby to lock the needle hub (30) in place and defeating the force of said partly compressed spring (40); wherein said gland assembly (20) incorporates a sliding activator sleeve (35), said spring (40) being in contact with the needle hub (30) and said sleeve (35), wherein engagement of said plunger (15) with said sleeve (35) forces the detent arrangement to release, allowing the spring (40) to push the needle hub (30) into said cavity in the plunger (15), **characterized in that** said gland assembly (20) incorporates said sliding activator sleeve (35) that is fitted around the needle hub (30) and said partly compressed spring (40); that the detent arrangement comprises one or more square or rectangular indent regions; and that the set of resilient claws (55) have complementary square or rectangular lugs that occupy the indent regions, wherein the upper region of said claws (55) have an inclined surface that is inclined away from the needle hub (30), and said sliding activator sleeve (35) is chamfered around its forward edge to produce an angled surface (90); and wherein, in assembly, this chamfered surface (90) is located as close as possible to the complementarily inclined surface (85) of said claws (55), such that, when said sleeve (35) is engaged by said plunger (15) moving in the direction of injection, the force applied thereby will tend to push the claws (55) away from the needle hub (30), thereby releasing it and allowing the spring (40) to push the needle hub (30) into the plunger (15).

2. The syringe (5) of claim 1, wherein the plunger (15) has at its liquid-engaging end a stopper (105), said stopper (105) being held in place by a detent arrangement (110) that may be overcome by the force of the spring pushing the needle hub (30) into the plunger (15).

3. The syringe (5) of claim 2, wherein the stopper (105) has a partly-spherical liquid-engaging surface (120).

## Patentansprüche

1. Automatisch einziehbare Spritze (5), die Folgendes aufweist: einen Zylinder (10), der einen offenen Vorderteil und einen in dem Zylinder (10) beweglichen Kolben (15) umfasst, eine Dichtungsdurchführungseinheit (20), die durch Presspassung oder Einschrauben in den offenen Vorderteil des Zylinders (10) eingesetzt ist, wobei die Dichtungsdurchführungseinheit (20) einen abnehmbaren Nadelansatz (30) umfasst, an dem eine Injektionsnadel (25) angebracht ist; wobei eine teilweise zusammengedrückte Feder (40) zwischen der Dichtungsdurchführungseinheit (20) und dem Nadelansatz (30) angeordnet ist, um dazu zu neigen, den Nadelansatz (30) in den Zylinder (10) zu drängen; wobei der Kolben (15, 215) einen inneren Hohlraum, der in der Lage ist, den Nadelansatz (30) aufzunehmen, und ein oder mehrere Löcher (210), um verdrängte Luft entweichen zu lassen, aufweist; wobei der Nadelansatz (30) vor dem Gebrauch durch eine Rastanordnung in Position gehalten wird, die die Bewegung des Nadelansatzes (30) sowohl in den als aus dem Zylinder (10) heraus verhindert; wobei die Rastanordnung einen oder mehrere Vertiefungsbereiche in dem Nadelansatz (30) umfasst, wobei der Vertiefungsbereich oder die Vertiefungsbereiche ein Profil aufweisen, dass, wenn es sich im Eingriff befände, die Bewegung des Nadelansatzes (30), ob in den oder aus dem Zylinder (10) heraus, verhindern würde; und einen Satz elastischer Klauen (55), die komplementäre Nasen aufweisen, die die Vertiefungsbereiche ausfüllen, um dadurch den Nadelansatz (30) in Position zu arretieren und die Kraft der teilweise zusammengedrückten Feder (40) zu überwinden; wobei die Dichtungsdurchführungseinheit (20) eine verschiebbare Auslöserhülse (35) einbezieht, wobei sich die Feder (40) mit dem Nadelansatz (30) und der Hülse (35) in Kontakt befindet, wobei der Eingriff des Kolbens (15) mit der Hülse (35) die Rastanordnung zum Loslassen zwingt, sodass der Feder (40) ermöglicht wird, den Nadelansatz (30) in den Hohlraum in dem Kolben (15) zu drücken,
**dadurch gekennzeichnet, dass** die Dichtungsdurchführungseinheit (20) die verschiebbare Auslöserhülse (35) einbezieht, die um den Nadelansatz (30) und die teilweise zusammengedrückte Feder (40) gepasst ist; dass die Rastanordnung einen oder mehrere quadratische oder rechteckige Vertiefungsbereiche umfasst; und dass der Satz elastischer Klauen (55) komplementäre quadratische oder rechteckige Nasen aufweisen, die die Vertiefungsbereiche ausfüllen, wobei der obere Bereich der Klauen (55) eine geneigte Oberfläche aufweist, die von dem Nadelansatz (30) weg geneigt ist, und die verschiebbare Auslöserhülse (35) um ihre Vorderkante abgeschrägt ist, um eine abgewinkelte Oberfläche (90) zu erzeugen; und wobei sich, wenn zusammengesetzt, diese abgeschrägte Oberfläche (90) möglichst nah an der komplementär geneigten Oberfläche (85) der Klauen (55) befindet, sodass, wenn der sich in der Richtung der Injektion bewegende Kolben (15) mit der Hülse (35) in Eingriff gelangt, die dadurch ausgeübte Kraft dazu neigt, die Klauen (55) von dem Nadelansatz (30) weg zu drücken, wodurch er freigegeben wird und die Feder (40) den Nadelansatz (30) in den Kolben (15) drücken kann.

2. Spritze (5) nach Anspruch 1, wobei der Kolben (15) an seinem Flüssigkeitseingriffsende einen Stopfen (105) aufweist, wobei der Stopfen (105) von einer Rastanordnung (110) in Position gehalten wird, die durch die Kraft der den Nadelansatz (30) in den Kolben (15) drückenden Feder überwunden werden kann.

3. Spritze (5) nach Anspruch 2, wobei der Stopfen (105) eine teilweise kugelförmige Flüssigkeitseingriffsoberfläche (120) aufweist.

## Revendications

1. Seringue auto-rétractable (5) ayant un corps (10) comprenant une partie avant ouverte, et un piston (15) mobile à l'intérieur dudit corps (10), un ensemble formant garniture (20) qui est inséré, par ajustement serré ou par vis, jusque dans la partie avant ouverte dudit corps (10), ledit ensemble formant garniture (20) logeant un raccord d'aiguille amovible (30) sur lequel une aiguille hypodermique (25) est attachée ; dans laquelle un ressort partiellement comprimé (40) est disposé entre l'ensemble formant garniture (20) et ledit raccord d'aiguille (30) de manière à tendre à solliciter le raccord d'aiguille (30) jusque dans ledit corps (10) ; dans laquelle ledit piston (15, 215) a une cavité interne en mesure de recevoir le raccord d'aiguille (30) et un ou plusieurs trous (210) pour permettre à l'air déplacé de s'échapper ; dans laquelle le raccord d'aiguille (30) est maintenu en place avant toute utilisation par un agencement à cliquet qui empêche tout mouvement du raccord d'aiguille (30) à la fois dans et hors du corps (10) ; dans laquelle l'agencement à cliquet comporte une ou plusieurs régions dentelées dans le raccord d'aiguille (30), lesdites région ou régions dentelées ayant un profil qui, quand il est en prise, permettrait d'empêcher tout mouvement dudit raccord d'aiguille (30), que ce soit dans ou hors du corps (10) ; et un jeu de griffes élastiques (55) qui ont des languettes complémentaires qui occupent les régions dentelées, pour de ce fait verrouiller le raccord d'aiguille (30) en place et vaincre la force dudit ressort partiellement comprimé (40) ; dans laquelle ledit ensemble formant garniture (20) incorpore un manchon activateur coulissant (35), ledit ressort (40) étant en contact avec le raccord d'aiguille (30) et ledit manchon (35), dans laquelle la mise en prise dudit piston (15) avec ledit manchon (35) force l'agencement à cliquet à se libérer, ceci permettant au ressort (40) de pousser le raccord d'aiguille (30) jusque dans ladite cavité dans le piston (15), **caractérisée en ce que** ledit ensemble formant garniture (20) incorpore ledit manchon activateur coulissant (35) qui est adapté autour du raccord d'aiguille (30) et dudit ressort partiellement comprimé (40) ; **en ce que** l'agencement à cliquet comporte une ou plusieurs régions dentelées carrées ou rectangulaire ; et **en ce que** les griffes du jeu de griffes élastiques (55) ont des languettes complémentaires carrées ou rectangulaires qui occupent les régions dentelées, dans laquelle la région supérieure desdites griffes (55) a une surface inclinée qui est inclinée à l'opposé du raccord d'aiguille (30), et ledit manchon activateur coulissant (35) est chanfreiné autour de son bord avant pour produire une surface inclinée (90) ; et dans laquelle, en assemblage, cette surface chanfreinée (90) est située aussi près que possible de la surface inclinée complémentaire (85) desdites griffes (55), de telle sorte que, quand ledit manchon (35) est mis en prise par ledit piston (15) se déplaçant dans la direction de l'injection, la force appliquée de ce fait va tendre à pousser les griffes (55) à l'opposé du raccord d'aiguille (30), pour de ce fait le libérer et permettre au ressort (40) de pousser le raccord d'aiguille (30) jusque dans le piston (15).

2. Seringue (5) selon la revendication 1, dans laquelle le piston (15) a, au niveau de son extrémité de mise en prise avec le liquide, une butée (105), ladite butée (105) étant maintenue en place par un agencement à cliquet (110) qui peut être vaincu par la force du ressort qui pousse le raccord d'aiguille (30) jusque dans le piston (15).

3. Seringue (5) selon la revendication 2, dans laquelle la butée (105) a une surface de mise en prise avec le liquide partiellement sphérique (120).
